# EUROPEAN PATENT SPECIFICATION

(11) **EP 2 692 863 B1**
(45) Date of publication and mention of the grant of the patent: **20.06.2018**
(21) Application number: 12765405.1
(22) Date of filing: 30.03.2012
(51) Int. Cl.: G01N 30/88, C12Q 1/68, G01N 30/26, C12N 15/00

(54) **PCR PRIMERS FOR PREPARING SAMPLES FOR SINGLE NUCLEOTIDE POLYMORPHISM DETECTION COMPRISING ALLELE-SPECIFIC PCR AND ION EXCHANGE CHROMATOGRAPHIC DETECTION**
PCR-PRIMER ZUR HERSTELLUNG VON PROBEN ZUR ERKENNUNG VON EINZELNUKLEOTID-POLYMORPHISMEN MITTELS ALLELE-SPEZIFISCHER PCR UND IONENAUSTAUSCH-CHROMATOGRAPHIE-ANALYSE.
AMORCES DE PCR POUR LA PREPARATION D'ECHANTILLONS POUR DETECTER DES POLYMORPHISMES MONONUCLEOTIDIQUES COMPRENANT UNE AMPLIFICATION PAR PCR SPECIFIQUE D'UN ALLELE ET ANALYSE DE LA DETECTION PAR CHROMATOGRAPHIE PAR ECHANGE D'IONS.

(30) Priority: 31.03.2011 JP 2011080369
(43) Date of publication of application: 05.02.2014
(73) Proprietor: Sekisui Medical Co., Ltd., Tokyo 103-0027 (JP)
(72) Inventor: YOTANI Takuya, Ryugasaki-shi Ibaraki 301-0852 (JP); KIYOTOH Eiji, Ryugasaki-shi Ibaraki 301-0852 (JP); USHIZAWA Koji, Tokyo 103-0027 (JP)
(74) Representative: Hart-Davis, Jason
(86) International application number: PCT/JP2012/058701
(87) International publication number: WO 2012/133834

(56) References cited:
- EP-A2- 0 507 591
- WO-A2-01/27331
- JP-A- 5 099 909
- JP-A- 2004 513 625
- JP-A- 2004 514 874
- JP-A- 2005 323 565
- MURIEL GAUDET ET AL: "Allele specific pcr in SNP genotyping", 1 January 2009 (2009-01-01), SINGLE NUCLEOTIDE POLYMORPHISMS : METHODS AND PROTOCOLS, HUMANA PR, USA, PAGE(S) 415 - 423, XP009177497, ISBN: 978-1-60327-410-4 * abstract; figures 26.1, 26.2 * * Item 3.1; page 419 *
- NEITZEL B ET AL: "EASY, ACCURATE AND RELIABLE SCREENING FOR SNPS BY ION PAIR/REVERSE PHASE HPLC: SIMULTANEOUS DETECTION OF FACTOR V G1691A, PROTHROMBIN G20210A AND METHYLENETETRAHYDROFOLATE REDUCTASE C677T VARIANTS", CLINICAL LABORATORY, CLIN LAB PUBLICATIONS, HEIDELBERG, vol. 49, no. 7/08, 1 January 2003 (2003-01-01), pages 313-318, XP008035171, ISSN: 1433-6510
- M. T. SEIPP ET AL: "Quadruplex Genotyping of F5, F2, and MTHFR Variants in a Single Closed Tube by High-Resolution Amplicon Melting", CLINICAL CHEMISTRY, vol. 54, no. 1, 16 November 2007 (2007-11-16), pages 108-115, XP055161525, ISSN: 0009-9147, DOI: 10.1373/clinchem.2007.097121
- THAYER J R ET AL: "Control of oligonucleotide retention on a pH-stabilized strong anion exchange column", ANALYTICAL BIOCHEMISTRY, ACADEMIC PRESS INC, NEW YORK, vol. 338, no. 1, 1 March 2005 (2005-03-01) , pages 39-47, XP004747515, ISSN: 0003-2697, DOI: 10.1016/J.AB.2004.11.013
- MATTHEW T. WEBSTER ET AL: "Analysis of variation in the human [beta]-globin gene cluster using a novel DHPLC technique", MUTATION RESEARCH/FUNDAMENTAL AND MOLECULAR MECHANISMS OF MUTAGENESIS, vol. 501, no. 1-2, 1 April 2002 (2002-04-01), pages 99-103, XP055161527, ISSN: 0027-5107, DOI: 10.1016/S0027-5107(02)00017-9
- GAUDET M. ET AL.: 'Allele-Specific PCR in SNP Genotyping' METHODS IN MOLECULAR BIOLOGY vol. 578, 2009, pages 415 - 424, XP009177497

## Description

### TECHNICAL FIELD

The present invention relates to a use of PCR primers for the preparation of samples for single nucleotide polymorphism detection in a method for preparing samples for single nucleotide polymorphism detection which are used in ion exchange chromatography analysis.

### BACKGROUND ART

In recent years, techniques have been developed for analyzing single nucleotide polymorphisms (SNP) which have been shown to be associated with various diseases and drug side effects; in the development thereof, it is an important factor to accurately detect single nucleotide polymorphisms simply and in a short time.

An RFLP (Restriction Fragment Length Polymorphism) method is known as a method for analyzing single nucleotide polymorphisms. The RFLP method involves, when a restriction enzyme exists recognizing a gene mutation site in a PCR (Polymerase Chain Reaction) amplification product, preparing primers in common sequence sites, performing amplification by holding polymorphisms in the PCR amplification product, cleaving the resultant PCR product with the restriction enzyme, and determining the presence or absence of polymorphisms based on the length of the fragments. However, the method has problems including that the use of restriction enzyme increases analysis cost and prolongs time of the whole analysis. It also has problems including that the detection of the chain length difference by electrophoresis complicates operation and prolongs time of the whole analysis.

In the fields of biochemistry, medicine, and the like, ion-exchange chromatography is used for the analysis of biomacromolecules such as nucleic acids, proteins, and polysaccharides as a method capable of accurately detecting them simply and in a short time. The use of ion-exchange chromatography reduces complicated operation as required for measurement by electrophoresis. Non Patent Literature 1 discloses a method for separating nucleic acid-related compounds by high-performance liquid chromatography. However, even the method disclosed in Non Patent Literature 1 has a problem that it is difficult to sufficiently separate nucleic acids having chain lengths approaching to each other such as single nucleotide polymorphisms.

Non Patent Literature 6 shows ion pair/reverse phase chromatography (HPLC).

Non Patent Literature 5 shows the use of a pH-stabilized strong anion exchange column for an analysis of single-strand oligonucleotides.

Non Patent Literature 3 and 4 teach the detection of single nucleotide polymorphisms with PCR amplification techniques.

### CITATION LIST

### - Patent Literature

Patent Literature 1: JP-A 2005-027518
Patent Literature 2: JP-A 2006-075126

WO 01/27331 A2 describes a method for separating heteroduplex DNA molecules in a mixture, wherein such a mixture is applied to an anion-exchange chromatography medium, the heteroduplex and homoduplex molecules are eluted with a mobile phase containing an eluting salt, including an anion and a cation, a buffer, and the eluting is carried out under conditions effective to at least partially denature the heteroduplexes. EP 0507591 A2 shows anion exchange chromatography of nucleic acids with alkylammonium salts.

### - Non Patent Literature

Non Patent Literature 1: *"*Raifusaiensu Notameno Kosoku Ekitai Kuromatogurafi Kiso To Jikken (High-Performance Liquid Chromatography for Life Science) (Basis and Experiment)", Hirokawa Shoten, p. 323, (1988)
Non Patent Literature 2: Nature, 324, pp. 163-166, (1986)
Non Patent Literature 3: Gaudet et al., "Allele-Specific PCR in SNP Genotyping", Chapter 26 in A.A. Komar (ed.), Single Nucleotide Polymorphisms, Methods in Molecular Biology 578, DOI 10.1007/978-1-60327-411-1_26, © Humana Press.
Non Patent Literature 4: Pattison et al., "Quadruplex Genotyping of F5, F2, and MTHFR Variants in a Single Closed Tube by High-Resolution Amplicon Melting", Clinical Chemistry 54:1 108 - 115 (2008).
Non Patent Literature 5: Thayer et al., "Control of oligonucleotide retention on a pH-stabilized strong anion exchange column", Analytical Biochemistry 338 (2005) 39 - 47. Non Patent Literature 6: Neitzel et al.: "Easy, Accurate and Reliable Screening for SNPs by Ion Pair/Reverse Phase HPLC: Simultaneous Detection of Factor V G1691A, Prothrombin G20210A and Methylenetetrahydrofolate _Reductase C677T Variants", Clin. Lab. 2003; 49:313 - 318.

### SUMMARY OF INVENTION

### - Technical Problem

Sample nucleic acids for single nucleotide polymorphism detection are for use in a simple method for quickly detecting a single nucleotide polymorphism. An object of the present invention is to provide a use of PCR primers for the preparation of samples for single nucleotide polymorphism detection in a method for preparing samples for single nucleotide polymorphism detection via ion exchange chromatography analysis.

### - Solution to Problem

The present invention is defined in the claims.The use of sample nucleic acids having specific features allows for quick and simple detection of single nucleotide polymorphisms.

The AS-PCR (Allele Specific-PCR) method is a method for detecting gene polymorphism (particularly, single nucleotide polymorphisms) using a sequence-specific amplification reaction. Specifically, PCR is performed in such a manner that a nucleotide sequence of a single nucleotide polymorphism desired to be detected is located at the 3' end of primer. When the sequence of the target nucleic acid is completely complementary to the primer, an extension reaction by DNA polymerase occurs . In contrast, when the sequence of the target nucleic acid is incompletely complementary to the primer, the extension reaction of DNA polymerase is inhibited. Thus, it is a method which involves using two primers, which have a wild-type or mutant-type nucleotide sequence of a single nucleotide polymorphism at the 3' end, to perform the determination of the single nucleotide polymorphism based on the results of the amplification reaction. The AS-PCR method can use a method as disclosed in Non Patent Literature 2.

In the present invention, AS-PCR is performed using primers having the following features.
(1) Two forward primers, specifically, a mutant-type forward primer and a wild-type forward primer are used. The mutant-type primer contains a single nucleotide polymorphic base at the 3' end, and the wild-type primer contains a wild-type base at the 3' end (corresponding to the single nucleotide polymorphism site).
(2) One of these two forward primers contains a 5'-end sequence that is incompletely complementary to a base sequence of a target nucleic acid (this sequence is also referred to as "tag sequence").
(3) The tag sequence has a chain length of 10 bp or less.
(4) These two forward primers are designed to amplify products with a chain length of 200 bp or less.
(5) The same reverse primer is used for both the mutant-type and wild-type.
(6) From the second cycle of the PCR amplification, in the presence of a PCR amplified product with the tag sequence at the 5' end derived from the mutant-type forward primer, a product with the tag sequence at the 3' end is amplified from the reverse primer.

The following generally describes the features of the PCR amplification in the case where the mutant-type primer, out of the above-mentioned primers, contains a tag sequence.
(A) The first cycle of the PCR amplification is characterized as follows. These two primers for the mutant-type and wild-type are annealed to a mutant-type template DNA and a wild-type template DNA, respectively, and extended to amplify a mutant-type DNA and a wild-type DNA, respectively. The full chain length of the amplified mutant-type DNA is longer than that of the amplified wild-type DNA by the chain length of the tag sequence.
(B) The second and later cycles of the PCR amplification are characterized as follows. The reverse primer is annealed to both the mutant-type DNA amplified product (containing the tag sequence at the 5' end) and the wild-type DNA amplified product, and extended to amplify both the mutant-type DNA and the wild-type DNA. The full chain length of the mutant-type DNA amplified from the reverse primer is also longer than that of the amplified wild-type DNA by the chain length of the tag sequence.

These two PCR products that differ from each other in full chain length by the chain length of the tag sequence are separated and analyzed by ion exchange chromatography as described below.

From the above general description, it will be appreciated by persons skilled in the art that both the mutant-type primer and the wild-type primer may contain a tag sequence as long as these two PCR products can be separated and analyzed by ion exchange chromatography, and that the tag sequence is not limited at all as long as the primers have essential features of general primers.

The terms "forward primer" and "reverse primer" as used herein have the normal meaning in the art. An embodiment in which the same forward primer is used for both the mutant-type and wild-type, and a reverse primer for the mutant-type out of reverse primers contains a tag sequence at the 5' end is also included within the scope of the present invention.

In conventional techniques, primers with such a tag sequence as that used in the present invention are used to avoid non-specific reactions (Patent Literature 1), or to allow for detection using a tag recognition probe (Patent Literature 2) . There has been no example of use of such probes for ion exchange chromatography samples, in particular, for samples for single nucleotide polymorphism detection.

Thus, the present invention provides the use of claim 1.

The method for detecting single nucleotide polymorphisms uses ion-exchange chromatography.

The eluent used for ion-exchange chromatography contains a guanidine salt derived from guanidine represented by formula (1) below.

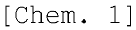

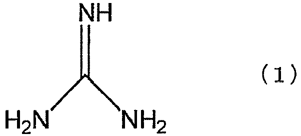

Examples of the guanidine salt include guanidine hydrochloride, guanidine sulfate, guanidine nitrate, guanidine carbonate, guanidine phosphate, guanidine thiocyanate, guanidine sulfamate, aminoguanidine hydrochloride, and aminoguanidine bicarbonate. Guanidine hydrochloride and guanidine sulfate are preferably used, among these.

The concentration of a guanidine salt in the eluent when analyzed may be properly adjusted in accordance with a substance to be detected; however, it is preferably 2,000 mmol/L or less.

Specifically, a method can be mentioned which involves performing gradient elution in the guanidine salt concentration range of 0 to 2,000 mmol/L. Thus, it is not necessary that the concentration of the guanidine salt in starting analysis is 0 mmol/L, and it is also not necessary that the concentration of the guanidine salt in terminating analysis is 2,000 mmol/L.

The method of gradient elution may be a low-pressure gradient method or a high-pressure gradient method; however, a method is preferable which involves carrying out elution while performing precise concentration adjustment by the high-pressure gradient method.

The guanidine salt may be added alone to the eluent or in combination with another salt. Examples of the salt capable of being used in combination with the guanidine salt include salts consisting of halides and alkali metals, such as sodium chloride, potassium chloride, sodium bromide, and potassium bromide, salts consisting of halides and alkali earth metals, such as calcium chloride, calcium bromide, magnesium chloride, and magnesium bromide, and inorganic acid salts such as sodium perchlorate, potassium perchlorate, sodium sulfate, potassium sulfate, ammonium sulfate, sodium nitrate, and potassium nitrate. Organic salts such as sodium acetate, potassium acetate, sodium succinate, and potassium succinate may also be used.

A known buffer or an organic solvent can be used as a buffer used in an eluent; specific examples thereof include Tris-hydrochloric acid buffer, TE buffer consisting of Tris and EDTA, TAE buffer consisting of Tris, acetic acid, and EDTA, and TBA buffer consisting of Tris, boric acid, and EDTA.

The pH of the eluent is not particularly limited, as long as it is in a range that allows the separation of nucleic acid chains by anionic exchange.

The filler used for ion-exchange chromatography is preferably one having cationic groups introduced into at least the surface of base material particles, and more preferably one having strong cationic groups and weak anionic groups on at least the surface of base material particles.

As used herein, the "strong cationic group" means a cationic group dissociating in the wide pH range of 1 to 14. Thus, the strong cationic group can retain a dissociated (cationized) state without being affected by the pH of the aqueous solution.

Examples of the strong cationic group include quaternary ammonium groups. Specific examples thereof include trialkylammonium groups such as a trimethylammonium group, a triethylammonium group, and a dimethylethylammonium group.

Examples of counter ions for the strong cationic group include halide ions such as chloride ion, bromide ion, and iodide ion.

The amount of the strong cationic group is not particularly limited; however, the lower limit thereof per dry weight of the filler is preferably 1 µeq/g and the upper limit is preferably 500 µeq/g. A strong cationic group amount of less than 1 µeq/g may weaken the retaining force of the filler and deteriorate separation performance. A strong cationic group amount of more than 500 µeq/g may pose problems of making the retaining force of the filler too strong, thereby not easily causing the elution of a substance, prolonging analysis time, and the like.

As used herein, the "weak anionic group" means an anionic group having a pKa of 3 or more. Thus, the weak anionic group described above is affected by the pH of the aqueous solution, by which the dissociated state thereof changes. A pH of more than 3 causes the dissociation of the proton of the carboxy group and increases the percentage thereof having a minus charge. Conversely, a pH of less than 3 increases the percentage of the carboxy group in an undissociated state in which the proton of the carboxy group is bonded.

Examples of the weak anionic group described above include a carboxy group and a phosphoric acid group. In particular, the weak anionic group is preferably a carboxy group.

Examples of methods for introducing carboxy groups into at least the surface of base material particles, which can be used, include known methods such as a method involving copolymerizing a carboxy group-containing monomer, a method involving hydrolyzing the ester moiety of a monomer, a method involving forming a carboxy group by ozonated water treatment, a method involving forming a carboxy group using ozone gas, a method involving forming a carboxy group by plasma treatment, a method involving reacting a carboxy group-containing silane coupling agent, and a method involving copolymerizing an epoxy group-containing monomer having and forming a carboxy group by ring-opening of the epoxy group. Among these, a method involving forming a carboxy group by ozonated water treatment is preferably used when the base material particle has hydrophobic structural portions, particularly carbon-carbon double bonds.

The method involving forming a carboxy group by ozonated water treatment will be described.

Ozone has high reactivity with a double bond, and the ozone reacting with the double bond forms ozonide as an intermediate, followed by the formation of a carboxy group and the like.

Ozonated water means what is formed by dissolving ozone gas in water.

Ozonated water can be used to simply oxidize the particle surface by merely dispersing the particles in the ozonated water. As a result, hydrophobic structural portions in the base material particle can be considered to be oxidized to form hydrophilic groups such as a carboxy group, a hydroxyl group, an aldehyde group, and a keto group.

Ozone has a strong oxidation effect; treatment with ozonated water is preferable because it can more uniformly oxidize the particle surface and causes the more uniform formation of carboxy groups than treatment with ozone gas.

The concentration of dissolved ozone in the ozonated water is not particularly limited; however, the lower limit thereof is preferably 20 ppm. A dissolved ozone concentration of less than 20 ppm requires a long time to form a carboxy group, or cannot sufficiently suppress the non-specific adsorption or the like of a substance to be detected since it causes the insufficient formation of a carboxy group. The lower limit of the dissolved ozone concentration is more preferably 50 ppm.

The ozonated water can be prepared, for example by a method involving contacting raw material water with ozone gas via an ozone gas-permeable membrane allowing only gas to pass therethrough and blocking the permeation of liquid as described, for example, in JP-A 2001-330969.

Under alkali conditions, it can be considered that the carboxy groups introduced into the surface of the base material particle are in a nearly dissociated state and produce weak cation exchange interaction with a few cations in a nucleic acid base.

It can also be considered that treatment with ozonated water causes the formation of hydrophilic groups such as a hydroxyl group, an aldehyde group, and a keto group in addition to a carboxy group and the presence of these hydrophilic groups weakens hydrophobic interaction acting between the filler surface and the nucleic acid.

Thus, it can be considered that the use of a filler having strong cationic groups and weak anionic groups on at least the surface improves separation performance by the action of the weak cation exchange interaction and the weakening of the hydrophobic interaction as described above in addition to the anion exchange interaction acting between the filler surface and a nucleic acid as the main interaction.

The amount of the weak anionic groups introduced into at least the surface of the base material particle is not particularly limited provided that it is smaller than or equal to the amount of the strong cationic group.

The base material particle which can be used is, for example, a synthetic polymer fine particle obtained using a polymerizable monomer or the like, and an inorganic fine particle such as silica; however, it is preferably one consisting of a hydrophobic cross-linked polymer particle consisting of an organic synthetic polymer and a layer consisting of a hydrophilic polymer having ion exchange groups copolymerized on the surface of the hydrophobic cross-linked polymer particle.

The hydrophobic cross-linked polymer may be a hydrophobic cross-linked polymer obtained by homopolymerizing one hydrophobic cross-linkable monomer, a hydrophobic cross-linked polymer obtained by copolymerizing two or more hydrophobic cross-linkable monomers, or a hydrophobic cross-linked polymer obtained by copolymerizing at least one hydrophobic cross-linkable monomer and at least one hydrophobic non-cross-linkable monomer.

The hydrophobic cross-linkable monomer is not particularly limited provided that it has 2 or more vinyl groups in one molecule of the monomer. Examples thereof include di(meth)acrylic esters such as ethylene glycol di(meth)acrylate, polyethylene glycol di(meth)acrylate, propylene glycol di(meth)acrylate, and polypropylene glycol di(meth)acrylate; tri(meth)acrylic esters or tetra(meth)acrylic esters such as tetramethylol methane tri (meth) acrylate, trimethylol propane tri (meth) acrylate, and tetramethylol methane tetra(meth)acrylate; and aromatic compounds such as divinylbenzene, divinyltoluene, divinylxylene, and divinylnaphthalene.

As used herein, the "(meth)acrylic" means "acrylic or methacrylic", and the "(meth)acrylate " means "acrylate or methacrylate".

The hydrophobic non-cross-linkable monomer is not particularly limited provided that it is a non-cross-linkable polymerizable organic monomer having hydrophobic properties; examples thereof include (meth)acrylic esters such as methyl(meth)acrylate, ethyl(meth)acrylate, propyl(meth)acrylate, isopropyl(meth)acrylate, butyl(meth)acrylate, and t-butyl(meth)acrylate, and styrene monomers such as styrene and methylstyrene.

When the hydrophobic cross-linked polymer consists of a copolymer of a hydrophobic cross-linkable monomer and a hydrophobic non-cross-linkable monomer, the lower limit of the content of the segment derived from the hydrophobic cross-linkable monomer in the hydrophobic cross-linked polymer is preferably 10% by weight, more preferably 20% by weight.

The hydrophilic polymer having ion exchange groups is composed of a hydrophilic monomer having an ion exchange group and shall contain the segment derived from a hydrophilic monomer having one or more kinds of ion exchange groups. Thus, Methods for producing a hydrophilic polymer having ion exchange groups include a method involving homopolymerizing a hydrophilic monomer having an ion exchange group and a method involving copolymerizing a hydrophilic monomer having an ion exchange group and a hydrophilic monomer not having an ion exchange group.

The hydrophilic monomer having an ion exchange group is preferably one having a strong cationic group and more preferably one having a quaternary ammonium group. Specific examples thereof include ethyl methacrylate trimethylammonium chloride, ethyl methacrylate triethylammonium chloride, ethyl methacrylate dimethylethylammonium chloride, ethyl acrylate trimethylammonium chloride, ethyl acrylate triethylammonium chloride, ethyl acrylate dimethylethylammonium chloride, acrylamide ethyltrimethylammonium chloride, acrylamide ethyltriethylammonium chloride, and acrylamide ethyldimethylethylammonium chloride.

The average particle diameter of the filler is not particularly limited; however, the preferable lower limit thereof is 0.1 µm, and the preferable upper limit is 20 µm. An average particle diameter of the filler of less than 0.1 µm increases the internal pressure of the column and may cause poor separation. An average particle diameter of the filler of more than 20 µm makes dead volume in the column too large and may cause poor separation.

As used herein, the average particle diameter refers to the volume average particle diameter, and can be measured using a particle size distribution analyzer (AccuSizer780 from Particle Sizing Systems).

According to the detection method for single nucleotide polymorphisms, the size of the product amplified by the AS-PCR method is preferably 200 bp or less. A size of the product amplified by the AS-PCR method of more than 200 bp may prolong amplification time of PCR and analysis time in ion-exchange chromatography or may cause insufficient separation performance. The size of the product amplified by the AS-PCR method is preferably 100 bp or less.

According to the detection method for single nucleotide polymorphisms, the size difference of the products (difference in chain length) between the wild-type and mutant-type amplified by the AS-PCR method is preferably 10 bp or less. When AS primers are designed so that the size difference of the products between the amplified wild-type and mutant-type exceeds 10 bp, desired amplification products may not be obtained due to a non-specific amplification reaction and the like.

Sample nucleic acids for single nucleotide polymorphism detection are for use in a simple method for quickly detecting single nucleotide polymorphisms. The present invention provides a use of PCR primers for the preparation of samples for single nucleotide polymorphism detection in a method for preparing samples for single nucleotide polymorphism detection via ion exchange chromatography analysis.

### BRIEF DESCRIPTION OF DRAWINGS

[Figure 1] Figure 1 is a pair of chromatograms obtained by separating and detecting wild-type 76 bp and mutant-type 79 bp in UGT1A1*6 region using anion exchange column 1 in Example 1.
[Figure 2] Figure 2 is a pair of chromatograms obtained by separating and detecting wild-type 76 bp and mutant-type 79 bp in UGT1A1*6 region using anion exchange column 2 in Example 1.
[Figure 3] Figure 3 is a pair of chromatograms obtained by separating and detecting wild-type 271 bp and mutant-type 274 bp in UGT1A1*6 region using anion exchange column 1 in Reference Example 1.
[Figure 4] Figure 4 is a pair of chromatograms obtained by separating and detecting wild-type 271 bp and mutant-type 274 bp in UGT1A1*6 region using anion exchange column 2 in Reference Example 1.
[Figure 5] Figure 5 is a pair of chromatograms obtained by separating and detecting wild-type 76 bp and mutant-type 79 bp in UGT1A1*6 region using anion exchange column 2 in Reference Example 2.

### DESCRIPTION OF EMBODIMENTS

Examples will be described below in further detail. However, the present invention is defined in the claims.

### (Provision of Anion Exchange Column)

### (Anion Exchange Column 1)

In a reactor provided with a stirrer, 300 g of tetraethylene glycol dimethacrylate (from Shin-Nakamura Chemical Co., Ltd.), 100 g of triethylene glycol dimethacrylate (from Shin-Nakamura Chemical Co., Ltd.), and 1.0 g of benzoyl peroxide (from Kishida Chemical Co., Ltd.) were added to 2,000 mL of aqueous solution of 3% by weight polyvinyl alcohol (from Nippon Synthetic Chemical Industry Co., Ltd.). The mixture was heated while stirring and polymerized at 80°C for 1 hour in an atmosphere of nitrogen. Then, 100 g of ethyl methacrylate trimethylammonium chloride (from Wako Pure Chemical Industries Ltd.) as a monomer having a strong cationic ion exchange group (a quaternary ammonium group) was dissolved in ion-exchange water, and the resultant solution was further added into the reactor. Subsequently, the solution was polymerized at 80°C for 2 hours in an atmosphere of nitrogen while stirring to provide a polymer composition. The resultant polymer composition was washed with water and acetone to provide hydrophilic coated polymer particles having quaternary ammonium groups on the surface of base material particles.

Ten (10) g of the resultant coated polymer particles were immersed in 300 mL of ozonated water having a dissolved ozone concentration of 100 ppm and stirred for 30 minutes. After stirring, centrifugation was performed using a centrifuge ("Himac CR20G" from Hitachi, Ltd.), and the supernatant was removed. This operation was repeated 2 times, and ozonated water treatment was applied to the coated polymer particles to provide a filler for ion-exchange chromatography in which quaternary ammonium groups and carboxy groups coexist.

The ozonated water was prepared using an ozonated water production system in which 400 hollow tube-shaped ozone gas permeable membranes 0.5 mm in inside diameter, 0.04 mm in thickness, and 350 cm in length were enclosed in a cylindrical mantle 15 cm in inside diameter and 20 cm in length (from Sekisui Chemical Co., Ltd.).

When the resultant filler for ion-exchange chromatography was measured using a particle size distribution analyzer ("Accusizer780" from Particle Sizing Systems), the average particle diameter thereof was found to be 10 µm.

The following column (anion exchange column 1) was provided using the resultant filler for ion-exchange chromatography.
Column size: 4.6 mm in inside diameter × 20 mm
Ion exchange group: quaternary ammonium group

### (Anion Exchange Column 2)

The following column as a commercially available column was provided.
Product name: TSK-gel DNA-STAT (from Tosoh Corporation)
Column size: 4.6 mm in inside diameter × 100 mm in length
Ion exchange group: quaternary ammonium group

### (Example 1)

The separation and detection of wild-type 76 bp and mutant-type 79 bp in UGT1A1*6 region was performed in Example 1.

### (AS-PCR amplification)

Wild-type and mutant-type amplification products were obtained using AS-PCR conditions as described below.

### (1) Reagent

AccuPrime Taq DNA Polymerase High Fidelity (from Invitorgen, Lot. 760816)
10 × AccuPrime PCR Buffer I
AccuPrime Taq DNA Polymerase High Fidelity (5 U/µL)
UGT1A1*6 primer (from Operon Biotechnologies)

Forward (wild-type) (10 µmol/µL):
5'-(cgcctcgttgtacatcagagcgg)-3' (SEQ ID NO: 1)

Forward (mutant-type) (10 pmol/µL):
5'-(ctgacgcctcgttgtacatcagagcga)-3" (SEQ ID NO: 2)

Reverse (10 pmol/(µL): 5'-(cacatcctccctttggaatggca)-3" (SEQ ID NO: 3)
Nuclease-free Water (not DEPC-treated) (from Ambion, Lot. 0803015)
UGT1A1 gene wild-type sequence-inserted plasmid (1 × 10⁶ copies/µL)
UGT1A1 gene mutant-type sequence-inserted plasmid (1 × 10⁶ copies/µL)

### (2) Preparation

One (1) µL of each UGT1A1 gene sequence-inserted plasmid was added to a solution prepared by adding Nuclease-free Water to 5 (µL of 10 × AccuPrime PCR Buffer I, 1 µL of the Forward primer, and 1 (µL of the Reverse primer to make a total volume of 49 µL of a reaction solution.

### (3) Reaction

PCR reaction was performed using C1000 (from BIO-RAD Laboratories). The temperature cycle is as described below.

The template was heat-degenerated at 94°C for 30 seconds; the amplification cycle of 94°C for 15 seconds, 62°C for 15 seconds, and 68°C for 30 seconds was repeated 40 times; and finally, incubation was carried out at 68°C for 5 minutes. The samples were stored at 4°C until use.

After AS-PCR amplification, bands derived from the amplification product were identified at about 80 bp by electrophoresis ("Mupid-ex" from Advance Co., Ltd.). The amplification product size was determined using 20 bp DNA Ladder Marker (from Takara Bio Inc.).

### (HPLC Analysis)

Using the provided anion exchange column, the AS-PCR amplification products were separated and detected under the following conditions.
System: LC-20A series (from Shimadzu Corporation)
Eluent: eluent A 25 mmol/L Tris-Hydrochloride buffer (pH 7.5)
   eluent B 25 mmol/L Tris-Hydrochloride buffer (pH 7.5) + 1 mol/L guanidine hydrochloride
Analysis time: Analysis time was 10 minutes when anion exchange column 1 was used.
   Analysis time was 20 minutes when anion exchange column 2 was used.
Elution method: the mixing ratio of eluent B was linearly increased using the following gradient conditions.
   Conditions when anion exchange column 1 was used
   0 minute (eluent B 40%) → 10 minutes (eluent B 50%) Conditions when anion exchange column 2 was used
   0 minute (eluent B 70%) → 20 minutes (eluent B 90%)
Analyte: Wild-type 76 bp in UGT1A1*6 region
   Mutant-type 79 bp in UGT1A1*6 region
Flow rate: 0.5 mL/min. (when anion exchange column 1 was used)
   1.0 mL/min. (when anion exchange column 2 was used)
Detection wavelength: 260 nm
Sample injection volume: 10 µL

### (Reference Example 1)

The separation and detection of wild-type 271 bp and mutant-type 274 bp in UGT1A1*6 region were performed in Reference Example 1.

### (AS-PCR Amplification)

Wild-type and mutant-type amplification products were obtained using the following AS-PCR conditions.

### (1) Reagent

AccuPrime Taq DNA Polymerase High Fidelity (from Invitrogen, Lot. 760816)
10 × AccuPrime PCR Buffer I
AccuPrime Taq DNA Polymerase High Fidelity (5 U/µL) UGT1A1*6 primer (from Operon Biotechnologies)

Forward (wild-type) (10 pmol/µL) :
5'-(cgcctcgttgtacatcagagcgg)-3' (SEQ ID NO: 1)

Forward (mutant-type) (10 pmol/µL) :
5'-(ctgacgcctcgttgtacatcagagcga)-3" (SEQ ID NO: 2)

Reverse (10 pmol/µL) : 5'-(gaaagggtccgtcagcatgac)-3" (SEQ ID NO: 4)
Nuclease-free Water (not DEPC-treated) (from Ambion, Lot. 0803015)
UGT1A1 gene wild-type sequence-inserted plasmid (1 × 10⁶ copies/µL)
UGT1A1 gene mutant-type sequence-inserted plasmid (1 × 10⁶ copies/µL)

### (2) Preparation

One (1) (µL of each UGT1A1 gene sequence-inserted plasmid was added to a solution prepared by adding Nuclease-free Water to 5 µL of 10 × AccuPrime PCR Buffer I, 1 µL of the Forward primer, and 1 µL of the Reverse primer to make a total volume of 49 µL of a reaction solution.

### (3) Reaction

PCR reaction was performed using C1000 (from BIO-RAD Laboratories). The temperature cycle is as described below.

The template was heat-degenerated at 94°C for 30 seconds; the amplification cycle of 94°C for 15 seconds, 62°C for 15 seconds, and 68°C for 30 seconds was repeated 40 times; and finally, incubation was carried out at 68°C for 5 minutes. The samples were stored at 4°C until use.

After AS-PCR amplification, bands derived from the amplification product were identified at about 270 bp (between 200 bp and 300 bp) by electrophoresis ("Mupid-ex" from Advance Co., Ltd.). The amplification product size was determined using 20 bp DNA Ladder Marker (from Takara Bio Inc.).

### (HPLC Analysis)

Using the provided anion exchange column, the AS-PCR amplification products were separated and detected under the following conditions.
System: LC-20A series (from Shimadzu Corporation)
Eluent: eluent A 25 mmol/L Tris-Hydrochloride buffer (pH 7.5)
   eluent B 25 mmol/L Tris-Hydrochloride buffer (pH 7.5) + 1 mol/L guanidine hydrochloride
Analysis time: Analysis time was 10 minutes when anion exchange column 1 was used.
   Analysis time was 20 minutes when anion exchange column 2 was used.
Elution method: the mixing ratio of eluent B was linearly increased using the following gradient conditions.
   Conditions when anion exchange column 1 was used
   0 minute (eluent B 60%) → 10 minutes (eluent B 80%) Conditions when anion exchange column 2 was used
   0 minute (eluent B 80%) → 20 minutes (eluent B 100%)
Analyte: Wild-type 271 bp in UGT1A1*6 region
   Mutant-type 274 bp in UGT1A1*6 region
Flow rate: 0.5 mL/min. (when anion exchange column 1 was used)
   1.0 mL/min. (when anion exchange column 2 was used)
Detection wavelength: 260 nm
Sample injection volume: 10 µL

### (Comparative Example 1)

The separation and detection of wild-type 76 bp and mutant-type 96 bp in UGT1A1*6 region were attempted to be performed in Comparative Example 1.

### (1) Reagent

AccuPrime Taq DNA Polymerase High Fidelity (from
Invitorgen, Lot. 760816)
10 × AccuPrime PCR Buffer I
AccuPrime Taq DNA Polymerase High Fidelity (5 U/µL) UGT1A1*6 primer (from Operon Biotechnologies)

Forward (wild-type) (10 pmol/µL):
5'-(cgcctcgttgtacatcagagcgg)-3' (SEQ ID NO: 1)

Forward (mutant-type) (10 pmol/µL):
5'-(atagttgtcctagcacctgacgcctcgttgtacatcagagcga)-3" (SEQ ID NO: 5)

Reverse (10 pmol/µL): 5'-(cacatcctccctttggaatggca)-3" (SEQ ID NO: 3)
Nuclease-free Water (not DEPC-treated) (from Ambion, Lot. 0803015)
UGT1A1 gene wild-type sequence-inserted plasmid (1 × 10⁶ copies/µL)
UGT1A1 gene mutant-type sequence-inserted plasmid (1 × 10⁶ copies/µL)

### (2) Preparation

One (1) µL of each UGT1A1 gene sequence-inserted plasmid was added to a solution prepared by adding Nuclease-free Water to 5 µL of 10 × AccuPrime PCR Buffer I, 1 µL of the Forward primer, and 1 µL of the Reverse primer to make a total volume of 49 µL of a reaction solution.

### (3) Reaction

PCR reaction was performed using C1000 (from BIO-RAD Laboratories). The temperature cycle is as described below.

The template was heat-degenerated at 94°C for 30 seconds; the amplification cycle of 94°C for 15 seconds, 62°C for 15 seconds, and 68°C for 30 seconds was repeated 40 times; and finally, incubation was carried out at 68°C for 5 minutes. The samples were stored at 4°C until use.

When, after AS-PCR amplification, the amplification product was determined by electrophoresis ("Mupid-ex" from Advance Co., Ltd.), many bands likely to indicate non-specific amplification were identified. This means that the AS-PCR amplification was not properly performed. Thus, HPLC analysis was not carried out.

### (Reference Example 2)

The separation and detection of wild-type 76 bp and mutant-type 79 bp in UGT1A1*6 region were performed in Reference Example 2.

HPLC analysis was performed using anion exchange column 2 in the same way as Example 1, except that the salt added to eluent B was sodium chloride in place of guanidine hydrochloride.

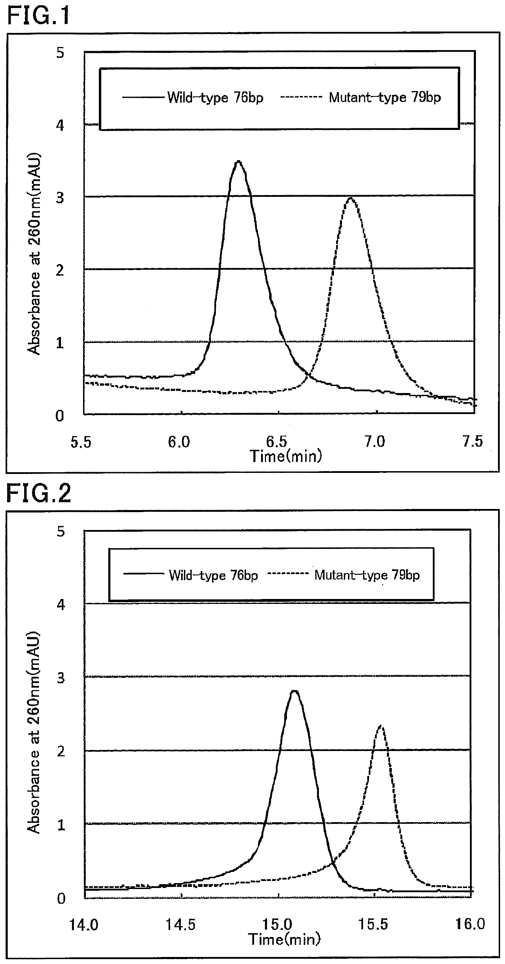

The chromatograms obtained by separating and detecting wild-type 76 bp and mutant-type 79 bp in UGT1A1*6 region in Example 1 are shown in Figure 1 (when anion exchange column 1 is used) and Figure 2 (when anion exchange column 2 is used) . The results of Figures 1 and 2 show that both columns could favorably separate and detect the wild-type 76 bp and mutant-type 79 bp in UGT1A1*6 region amplified by AS-PCR. Particularly, the use of anion exchange column 1 could almost completely separate and detect them in a short time.

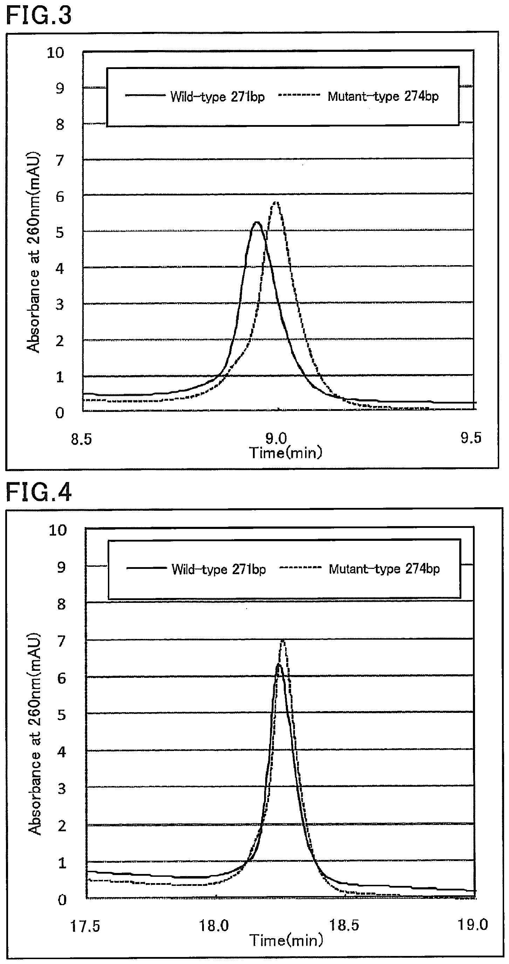

The chromatograms obtained by separating and detecting wild-type 271 bp and mutant-type 274 bp in UGT1A1*6 region in Reference Example 1 are shown in Figure 3 (when anion exchange column 1 is used) and Figure 4 (when anion exchange column 2 is used) . The results of Figures 3 and 4 show that the wild-type 271 bp and mutant-type 274 bp in UGT1A1*6 region amplified by AS-PCR could not be separated in contrast to Example 1. It can be considered that the reason for this lies in the fact that, compared to the size of the AS-PCR amplification products, the difference in the chain length between the wild-type and the mutant-type was relatively small.

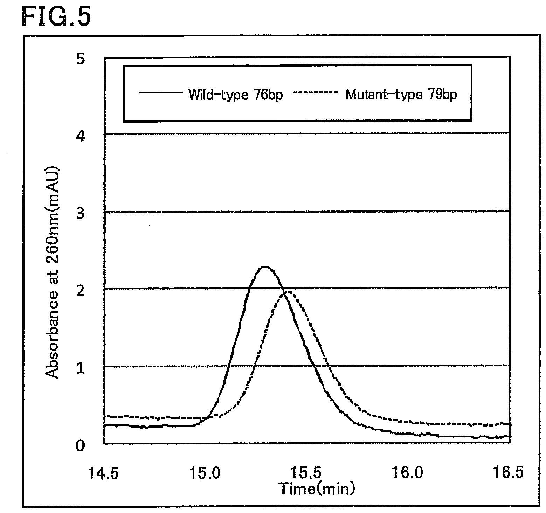

The chromatograms obtained by separating and detecting wild-type 76 bp and mutant-type 79 bp in UGT1A1*6 region in Reference Example 2 are shown in Figure 5. When sodium chloride was added in place of guanidine hydrochloride to the eluent B, the wild-type 76 bp and the mutant-type 79 bp could not be separated.

### INDUSTRIAL APPLICABILITY

Sample nucleic acids for single nucleotide polymorphism detection are for use in a simple method for quickly detecting single nucleotide polymorphisms. The present invention provides a use of PCR primers for the preparation of samples for single nucleotide polymorphism detection in a method for preparing samples for single nucleotide polymorphism detection via ion exchange chromatography analysis.

## Claims

1. Use of PCR primers for preparation of a nucleic acid sample for single nucleotide polymorphism detection using AS-PCR, wherein the primers have the following features:
(a) two forward primers are designed to amplify together with a common reverse primer a product having a full chain length of 200 bp or less by AS-PCR, which two forward primers consist of
-- a mutant type forward primer containing a single nucleotide base at the 3'-end and
-- a wild-type forward primer containing a wild-type base at the 3'-end, which corresponds to the single nucleotide polymorphism site,
(b) one of the forward primers has a tag sequence incompletely complementary to a base sequence of a target nucleic acid at the 5' end; and
(c) the tag sequence has a chain length of 10 bp or less,
in a method for preparing said sample for single nucleotide polymorphism detection using AS-PCR,
**characterized in that**
ion exchange chromatography analysis with an eluent comprising a guanidine salt and a filler comprising cationic groups dissociating in the pH range of 1 to 14 and anionic groups having a pKa of 3 or more on at least the surface of base material particles is used for the detection of the AS-PCR amplification products from an elution with said eluent.

## Patentansprüche

1. Verwendung von PCR Primern zur Herstellung einer Nukleinsäureprobe zur Einzelnukleotid-Polymorphismusdetektion unter Verwendung von AS-PCR, wobei die Primer folgende Merkmale haben:
(a) zwei Forward-Primer sind entworfen, um zusammen mit einem gemeinsamen Reverse-Primer ein Produkt mit einer vollen Kettenlänge von 200 bp oder weniger durch AS-PCR zu vervielfältigen, welche zwei Forward-Primer bestehen aus
-- einem Forward-Primer vom Mutantentyp, der eine Einzelnukleotidbase am 3'-Ende enthält und
-- einem Forward-Primer vom Wildtyp, der eine Wildtyp Base am 3'-Ende enthält, welche der Einzelnukleotid-Polymorphismusstelle entspricht,
(b) einer der Forward-Primer hat eine anhängende Sequenz, die unvollständig komplementär zu einer Basesequenz einer Zielnukleinsäure am 5'-Ende ist, und
(c) die anhängende Sequenz hat eine Kettenlänge von 10 bp oder weniger,
in einem Verfahren zur Herstellung der Probe zur Einzelnukleotid-Polymorphismusdetektion unter Verwendung von AS-PCR,
**dadurch gekennzeichnet, dass**
Ionenaustauschchromatographieanalyse mit einem Elutionsmittel, umfassend ein Guanidin-Salz und einen Füllstoff, der kationische Gruppen enthält, die im pH-Bereich von 1 bis 14 dissoziieren und anionische Gruppen mit einem pKa von 3 oder mehr an wenigstens einer Oberfläche der Basismaterialpartikel, verwendet wird zur Detektion der AS-PCR Vervielfältigungsprodukte aus einer Elution mit dem Elutionsmittel.

## Revendications

1. Utilisation d'amorces PCR pour la préparation d'un échantillon d'acide nucléique pour une détection de polymorphisme à nucléotide simple utilisant AS-PCR, dans laquelle les amorces présentent les caractéristiques suivantes :
(a) deux amorces sens sont désignées pour amplifier ensemble avec une amorce anti-sens un produit ayant une longueur complète de chaîne de 200 bp ou inférieure par AS-PCR, lesquelles deux amorces sens consistent en
-- une amorce sens de type mutant contenant une base de nucléotide simple à l'extrémité 3' et
-- une amorce sens de type sauvage contenant une base de type sauvage à l'extrémité 3', qui correspond au site de polymorphisme à nucléotide simple,
(b) une des amorces sens présente une séquence de marqueur incomplètement complémentaire à une séquence de base d'un acide nucléique cible à l'extrémité 5' ; et
(c) la séquence de marqueur présente une longueur de chaîne de 10 bp ou inférieure,
dans un procédé de préparation dudit échantillon pour une détection de polymorphisme à nucléotide simple utilisant AS-PCR,
**caractérisée en ce que**
une analyse de chromatographie à échange d'ions avec un éluant comprenant un sel de guanidine et une charge comprenant des groupes cationiques se dissociant dans l'intervalle de pH de 1 à 14 et des groupes anioniques ayant un pKa de 3 ou supérieur sur au moins la surface de particules de matériau de base est utilisée pour la détection des produits d'amplification AS-PCR d'une élution avec ledit éluant.


### SEQUENCE LISTING

<110> SEKISUI MEDICAL CO., LTD.
<120> SAMPLE NUCLEIC ACID FOR SINGLE NUCLEOTIDE POLYMORPHISM DETECTION
   PURPOSES, PCR PRIMER FOR PREPARING SAMPLE FOR SINGLE NUCLEOTIDE POLYMORPHISM DETECTION PURPOSES, AND METHOD FOR PREPARING SAMPLE FOR SINGLE NUCLEOTIDE POLYMORPHISM DETECTION PURPOSES WHICH CAN BE USED IN ION EXCHANGE CHROMATOGRAPHIC ANALYSIS
<130> S-1388
<150> JP 2011-080369
   <151> 2011-03-31
<160> 5
<210> 1
   <211> 23
   <212> DNA
   <213> Artificial Sequence
<220>
   <223> Description of Artificial Sequence: primer
<400> 1
   cgcctcgttg tacatcagag cgg 23
<210> 2
   <211> 27
   <212> DNA
   <213> Artificial Sequence
<220>
   <223> Description of Artificial Sequence: primer
<400> 2
   ctgacgcctc gttgtacatc agagcga 27
<210> 3
   <211> 23
   <212> DNA
   <213> Artificial Sequence
<220>
   <223> Description of Artificial Sequence: primer
<400> 3
   cacatcctcc ctttggaatg gca 23
<210> 4
   <211> 21
   <212> DNA
   <213> Artificial Sequence
<220>
   <223> Description of Artificial Sequence: primer
<400> 4
   gaaagggtcc gtcagcatga c 21
<210> 5
   <211> 43
   <212> DNA
   <213> Artificial Sequence
<220>
   <223> Description of Artificial Sequence: primer
<400> 5
   atagttgtcc tagcacctga cgcctcgttg tacatcagag ga 43